# EUROPÄISCHE PATENTSCHRIFT

(11) **EP 1 555 039 B1**
(45) Veröffentlichungstag und Bekanntmachung des Hinweises auf die Patenterteilung: **30.08.2006**
(21) Anmeldenummer: 04024718.1
(22) Anmeldetag: 16.10.2004
(51) Int. Cl.: A61M 16/06

(54) **Atemmaske mit Stirnstütze**
Respiratory mask with forehead support
Masque respiratoire avec support frontal

(30) Priorität: 19.01.2004 DE 102004002870
(43) Veröffentlichungstag der Anmeldung: 20.07.2005
(73) Patentinhaber: Weinmann Geräte für Medizin GmbH & Co. KG, 22525 Hamburg (DE)
(72) Erfinder: Frerichs, Arnold, 21614 Buxtehude (DE); Schulz, Gerd, 22869 Schenefeld (DE)
(74) Vertreter: Klickow, Hans-Henning

(56) Entgegenhaltungen:
- EP-A- 1 334 742
- WO-A-03/082406
- US-B1- 6 530 373

## Beschreibung

Die Erfindung betrifft eine Atemmaske, die einen Maskengrundkörper, einen Schlauchanschluß sowie mindestens ein Dichtelement aufweist und die mit einer Stirnstütze versehen ist, die relativ zum Maskengrundkörper positionierbar angeordnet ist, sowie bei der ein die Stirnstütze mit dem Maskengrundkörper koppelndes Verbindungselement relativ zu einer Führung derart verschieblich angeordnet ist, daß das Verbindungselement vorgebbar weit aus der Führung herausragt und relativ zur Führung in mindestens zwei unterschiedlichen Positionierungen arretierbar ist.

Derartige Atemmasken können sowohl als Nasalmasken als auch als Vollgesichtsmasken ausgebildet sein. Typischerweise verfügen derartige Atemmasken über eine bewegliche Stirnstütze, die eine optimale Positionierung der Maske auf un= terschiedlich geformten Gesichtern der jeweiligen Patienten unterstützt. Üblicherweise erfolgt die Bewegung der Stirnstütze relativ zum Maskengrundkörper durch ein Schwenken um eine Querachse oder durch ein Schwenken um eine senkrechte Achse.

Nachteilig sowohl beim Schwenken der Stirnstütze relativ zu einer Querachse als auch beim Schwenken um eine senkrechte Achse herum ist es, daß derartige Bewegungen nur indirekt den Abstand zwischen der Stirnstütze und dem Maskengrundkörper festlegen.

Bekannt ist es darüber hinaus, eine Verstellung der Stirnstütze relativ zum Maskengrundkörper entsprechend der DE 101 55 152 unter Verwendung von Stangen vorzunehmen. Die Verwendung derartiger Stangen oder die Verwendung von Schrauben führt aber zu Integrationsproblemen bei der Einführung dieser Bauelemente in die Maskengeometrie.

Aus der WO 03/082406 ist es bekannt, eine Stirnstütze unter Verwendung eines gebogenen Verbindungselementes relativ zu einem Maskengrundkörper zu positionieren. Das Verbindungselement ist in einer ebenfalls gebogen verlaufenden Schiene geführt. Durch ein unterschiedlich weites Herausziehen des gebogenen verbindungselementes aus der gebogenen Schiene erfolgt eine Abstandsveränderung der Stirnstütze relativ zum Maskengrundkörper. Zusätzlich zu einer Translationsbewegung der Stirnstütze relativ zum Maskengrundkörper erfolgt durch das Herausziehen des verbindungselementes aus der Führungsschiene ebenfalls ein Verkippen der Stirnstütze relativ zum Maskengrundkörper sowie eine veränderung der Höhenpositionierung. Das Dokument WO 03/082406 wird als nächstliegender Stand der Technik angesehen.

Aufgabe der vorliegenden Erfindung ist es, eine Atemmaske der einleitend genannten Art derart zu konstruieren, daß eine Verstelleinrichtung für die Stirnstütze sowohl eine hohe Funktionalität aufweist als auch in einfacher Weise in die geometrische Gestaltung des Maskengrundkörpers integriert werden kann.

Diese Aufgabe wird erfindungsgemäß dadurch gelöst, daß das Verbindungselement flexibel ausgebildet ist.

Die verschiebliche Anordnung des Verbindungselementes in der Führung derart, daß in Abhängigkeit von der jeweiligen Arretierung ein unterschiedlich weites Herausragen aus der Führung realisiert ist, ermöglicht eine direkte Verstellbarkeit der Atemmaske auf dem Gesicht des Patienten. Durch die flexible Ausbildung des Verbindungselementes ist es möglich, eine Integration in die gerundete Kontur des Maskengrundkörpers vorzunehmen.

Eine spritzgußtechnische Herstellung der verwendeten Bauteile wird dadurch unterstützt, daß das Verbindungselement bandartig ausgebildet ist.

Gemäß einer anderen Ausführungsform ist auch daran gedacht, daß das Verbindungselement spiralartig ausgebildet ist.

Darüber hinaus ist es auch möglich, daß das verbindungselement als eine mit einem steifen Endstück versehene Gliederkette ausgebildet ist.

Eine Anpassung an einen typischen Formverlauf einer Atemmaske erfolgt dadurch, daß die Führung eine gekrümmte Führungsbahn für das Verbindungselement aufweist.

Eine einfache mechanische Realisierung kann dadurch erreicht werden, daß ein Arretierelement zur Arretierung des Verbindungselementes relativ zur Führung mindestens einen Vorsprung und mindestens eine Ausnehmung aufweist.

Eine gute Bedienbarkeit wird dadurch unterstützt, daß das Verbindungselement eine Bedientaste aufweist.

Eine konstruktiv einfache Realisierung unter Nutzung von Biegespannungen innerhalb des Verbindungselementes kann dadurch erfolgen, daß das Verbindungselement relativ zur Führung durch eine federnde Verspannung arretierbar ist.

Eine erhöhte mechanische Stabilität sowie eine verbesserte Führungsgenauigkeit können dadurch erreicht werden, daß das Verbindungselement zwei relativ zueinander im wesentlichen parallel verlaufende Bänder aufweist.

Insbesondere ist daran gedacht, daß eines der Bänder zur Führung und das andere der Bänder zur Abstützung des Verbindungselementes ausgebildet ist.

Eine direkte Verstellmöglichkeit der Stirnstütze auf dem Gesicht des Patienten wird insbesondere dadurch erreicht, daß ein aus der Führung herausragendes Ende des Verbindungselementes im wesentlichen senkrecht zu einer Grundebene des Maskengrundkörpers verläuft.

Eine Fixierung des der Stirnstütze abgewandten Endes des Verbindungselementes kann dadurch erfolgen, daß das Verbindungselement im Bereich seines der Stirnstütze abgewandten Endes mindestens eine an einen Fixierzapfen angepaßte Fixierausnehmung aufweist.

Eine Fixierung des Verbindungselementes in mindestens zwei unterschiedlichen Fixierungspositionen kann dadurch erreicht werden, daß das Verbindungselement mindestens zwei Fixierausnehmungen aufweist.

In den Zeichnungen sind Ausführungsbeispiele der Erfindung schematisch dargestellt. Es zeigen:
- Fig. 1: Eine perspektivische Darstellung eines Beamtungsgerätes mit Verbindungsschlauch zu einer Beatmungsmaske,
- Fig. 2: eine perspektivische Darstellung einer Atemmaske mit Stirnstütze, bei der ein Verbindungselement zwischen der Stirnstütze und einem Maskengrundkörper flexibel ausgebildet und in einer Schiene geführt ist,
- Fig. 3: eine perspektivische Darstellung einer anderen Atemmaske, bei der das Verbindungselement zwischen der Stirnstütze und dem Maskengrundkörper aus zwei mit einem Abstand parallel zueinander geführten flexiblen Bändern ausgebildet ist,
- Fig. 4: eine weitere Darstellung der Atemmaske gemäß Fig. 3,
- Fig. 5: ein Querschnitt gemäß Schnittlinie V-V in Fig. 4,
- Fig. 6: eine Ausführungsform, bei der das Verbindungselement blattfederartig ausgebildet und in einer Führung arretiert ist und
- Fig. 7: eine gegenüber Fig. 6 abgewandelte Ausführungsform mit zweifacher verstellbarer Fixierung des Verbindungselementes.

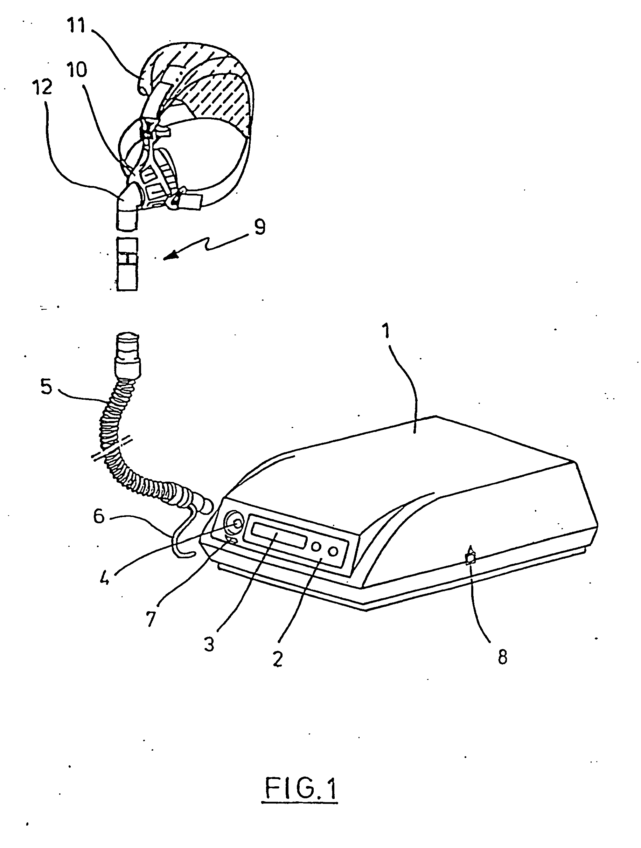

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Atemgasschlauch (5) angeschlossen. Entlang des Atemgasschlauches (5) kann ein zusätzlicher Druckmeßschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung des Atemgasschlauches (5) ist ein Ausatmungselement (9) angeordnet. Ebenfalls kann ein Ausatemventil verwendet werden.

Fig. 1 zeigt darüber hinaus eine Atemmaske (10), die als Nasalmaske ausgebildet ist. Eine Fixierung im Bereich eines Kopfes eines Patienten kann über eine Kopfhaube (11) erfolgen. Im Bereich ihrer dem Atemgasschlauch (5) zugewandten Ausdehnung weist die Atemmaske (10) eine Schlauchkupplung (12) auf.

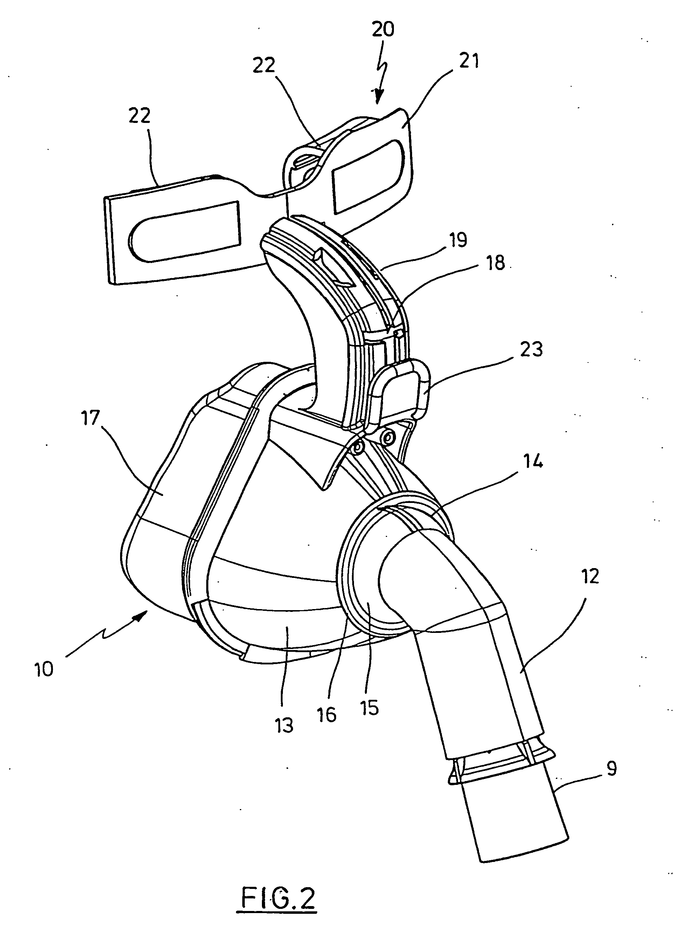

Aus Fig. 2 ist der Aufbau der Atemmaske (10) im Detail zu erkennen. Die Schlauchkupplung (12) für den nicht dargestellten Atemgasschlauch (5) ist mit einem Maskengrundkörper (13) der Atemmaske (10) über ein Gelenk (14) verbunden, das kugelgelenkartig ausgebildet ist. Beim dargestellten Ausführungsbeispiel besteht das Gelenk (14) aus einem mit der Schlauchkupplung (12) verbundenen Innenteil (15) sowie einer mit dem Maskengrundkörper (13) verbundenen Außenschale (16). Das Innenteil (15) ist wenigstens bereichsweise kugelsegmentartig ausgebildet und die Außenschale (16) erstreckt sich entlang wenigstens eines Teiles der kugelsegmentartigen Oberfläche des Innenteiles (15).

Am Maskengrundkörper (13) ist zur Abdichtung relativ zu einem Gesicht eines Patienten ein Dichtelement (17) angeordnet, das mindestens bereichsweise als Lippendichtung ausgebildet ist. Über ein Verbindungselement (18), das mindestens bereichsweise von einer Führung (19) gehaltert ist, wird eine Stirnstütze (20) mit dem Maskengrundkörper (13) gekoppelt. Die Stirnstütze (20) weist einen Träger (21) auf, der ein Stirnpolster (22) haltert.

Bei dem in Fig. 2 dargestellten Ausführungsbeispiel ist die Führung (19) ausgehend vom Maskengrundkörper (13) in eine von der Schlauchkupplung (12) wegweisende Richtung gekrümmt ausgebildet. Wenigstens bereichsweise innerhalb der Führung (19) verläuft das Verbindungselement (18), das flexibel und bandartig konstruiert ist. Das Verbindungselement (18) ist innerhalb der Führung (19) in mindestens zwei unterschiedlichen Positionierungen arretierbar. Eine Arretierung sowie eine Positionierung des Verbindungselementes (18) erfolgt unter Verwendung einer Bedientaste (23). Beim Drücken der Bedientaste (23) wird die Arretierung gelöst und das Verbindungselement (18) kann innerhalb der Führung (19) verschoben werden. Nach einem Loslassen der Bedientaste (23) erfolgt eine erneute Arretierung des Verbindungselementes (18).

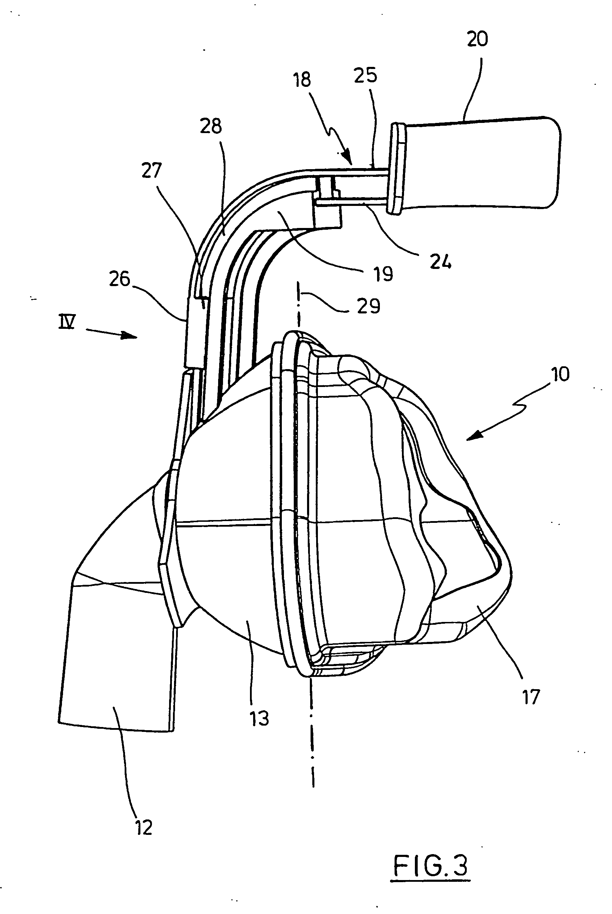

Bei der in Fig. 3 dargestellten Ausführungsform ist das Verbindungselement (18) aus zwei relativ zueinander im wesentlichen parallel verlaufenden Bändern (24, 25) ausgebildet. Das erste Band (24) ist innerhalb der Führung (19) positionierbar, das zweite Band (25) verläuft entlang einer äußeren Begrenzung der Führung (19). Das Band (25) weist im Bereich eines Endstückes (26) Seitenstege (27) auf, die in Seitennuten der Führung (19) eingreifen. Über das Endstück (26) kann ein Verschieben des Verbindungselementes (18) erfolgen. Das Band (24) dient im wesentlichen zur Abstützung.

Fig. 3 veranschaulicht, daß bei einer Verschiebung des Verbindungselementes (18) relativ zur Führung (19) eine Positionierung der Stirnstütze (20) im wesentlichen senkrecht zu einer Grundebene (29) der Atemmaske (10) erfolgt. Diese Anordnung unterstützt die direkte Positionsvorgabe der Stirnstütze (20).

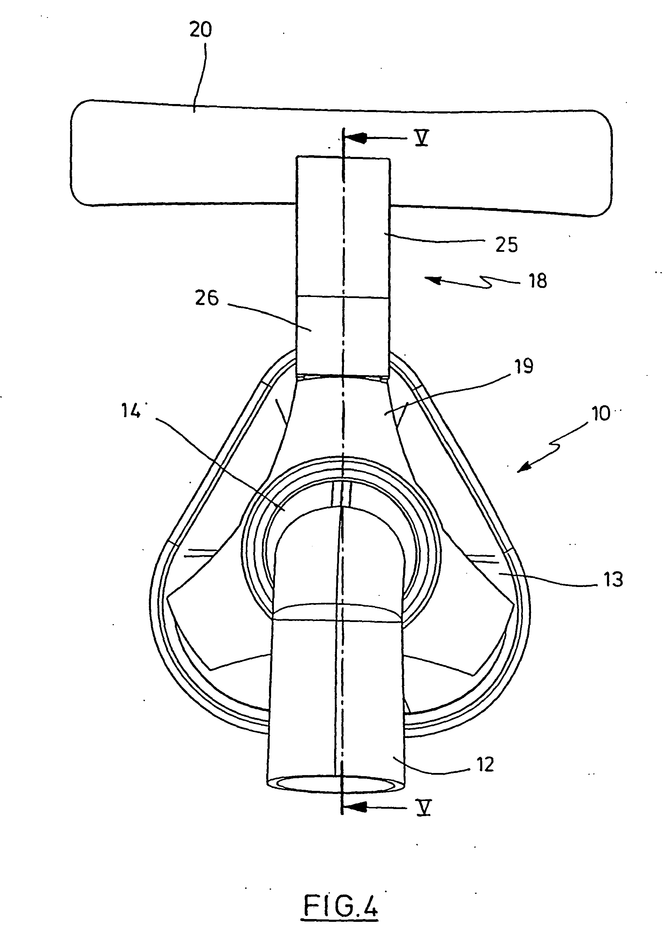

Fig. 4 zeigt den Aufbau der Atemmaske (10) gemäß Fig. 3 entsprechend Blickrichtung IV in Fig. 3. Zu erkennen ist insbesondere der im wesentlichen symmetrische Aufbau relativ zu der in Fig. 4 senkrecht verlaufenden Mittelebene.

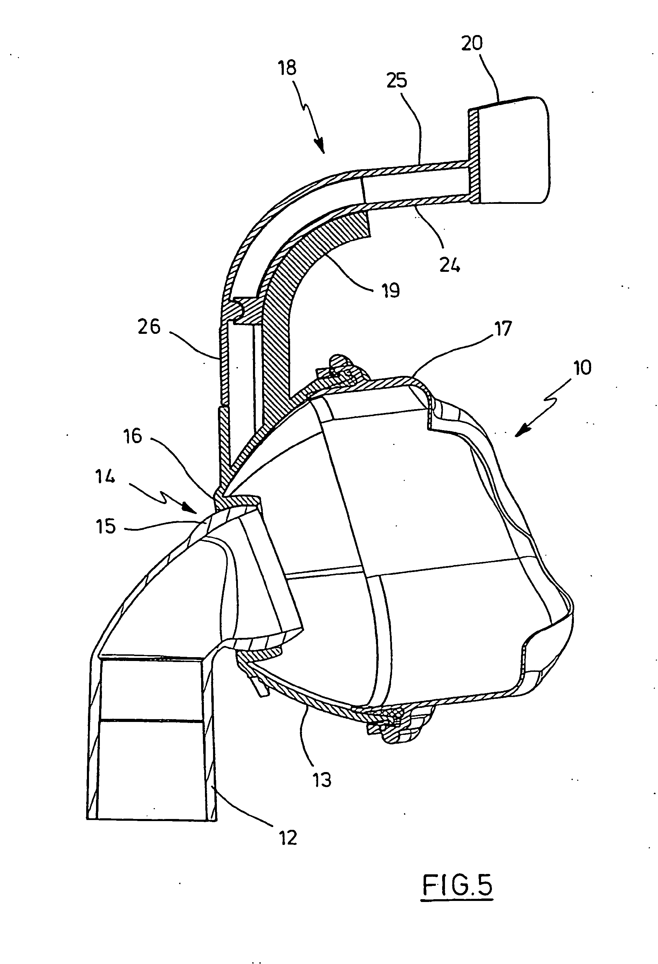

Fig. 5 veranschaulicht einen Vertikalschnitt durch die Atemmaske (10) gemäß Fig. 4. Zu erkennen ist beispielsweise, daß die Bänder (24, 25) innerhalb der Führung von einem Querelement (30) relativ zueinander abgestützt sind. Das Querelement (30) ist zweiteilig von Vorsprüngen (31, 32) ausgebildet, die sich jeweils ausgehend von den Bändern (24, 25) in einander zugewandte Richtungen erstrecken und eine aneinander angepaßte Formgebung aufweisen, so daß die Vorsprünge (31, 32) bereichsweise ineinander eingreifen.

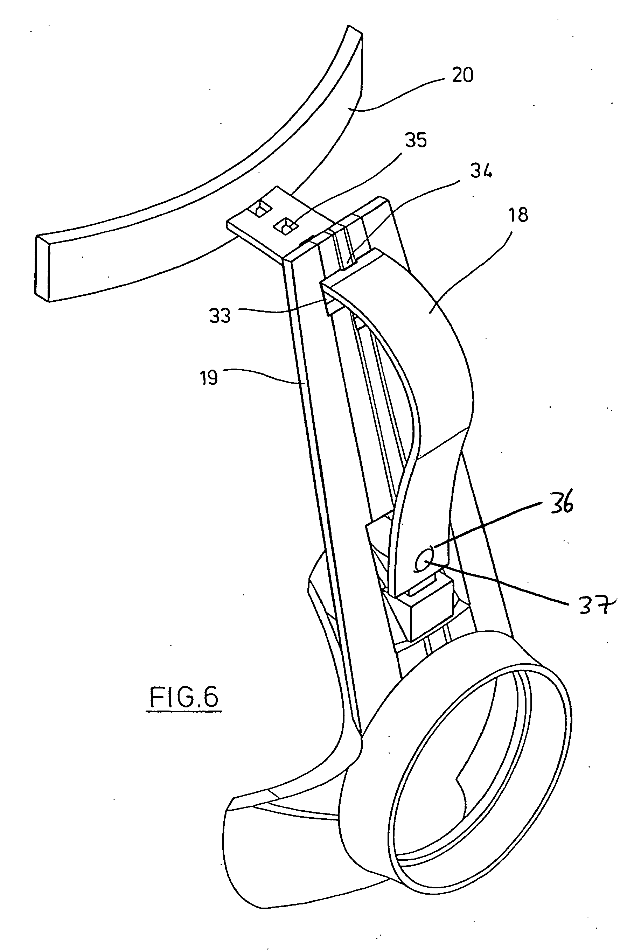

Fig. 6 zeigt eine Ausführungsform, bei der die Führung (19) separat und zur Verbindung mit einem nicht dargestellten Maskengrundkörper (13) ausgebildet ist. Das Verbindungselement (18) ist bandartig und mit einer Eigenspannung ähnlich einer Blattfeder realisiert. Das Verbindungselement (18) erstreckt sich dabei durch eine Ausnehmung (33) der Führung (19) hindurch. Im Bereich der Ausnehmung (33) ist ein als Teil der Führung (19) ausgebildeter Vorsprung (34) angeordnet, der bei einer entsprechenden Positionierung des Verbindungselementes (18) in eine Ausnehmung (35) des Verbindungselementes (18) eingreift und hierdurch eine Arretierung des Verbindungselementes (18) relativ zur Führung (19) vornimmt.

In einer Längsrichtung des Verbindungselementes (18) sind mehrere Ausnehmungen (35) hintereinander angeordnet, so daß in Abhängigkeit von der Auswahl der jeweiligen Arretierungspositionierung das Verbindungselement (18) unterschiedlich weit aus der Ausnehmung (25) heraussteht und hierdurch unterschiedliche Abstände der Stirnstütze (20) relativ zum Maskengrundkörper (13) realisiert.

Grundsätzlich ist es möglich, die in Fig. 6 schematisch dargestellte Konstruktion mit einer Verkleidung im Bereich des Verbindungselementes (18) zu versehen, so daß eine harmonische Konturüberleitung zu einer Gestaltung des Maskengrundkörpers (13) gewährleistet ist. Eine Fixierung des Verbindungselementes (18) innerhalb der Ausnehmung (33) erfolgt aufgrund der blattfederartigen Spannung innerhalb des Verbindungselementes (18), die aus dem in Fig. 6 erkennbaren gebogenen Verlauf des Verbindungselementes (18) resultiert.

Gemäß der Ausführungsform in Fig. 6 ist das Verbindungselement (18) im Bereich seines der Ausnehmung (33) abgewandten Endes mit eine Fixierausnehmung (36) versehen, die einen Fixierzapfen (37) umschließt, der beim dargestellten Ausführungsbeispiel als Teil der Führung (19) ausgebildet ist. Zur Verhinderung eines ungewollten Trennens des Verbindungselementes (18) und des Fixierzapfens (37) ist insbesondere daran gedacht, den Fixierzapfen (37) in einem aus der Fixierausnehmung herausragenden Bereich mit einer Randverdickung zu versehen.

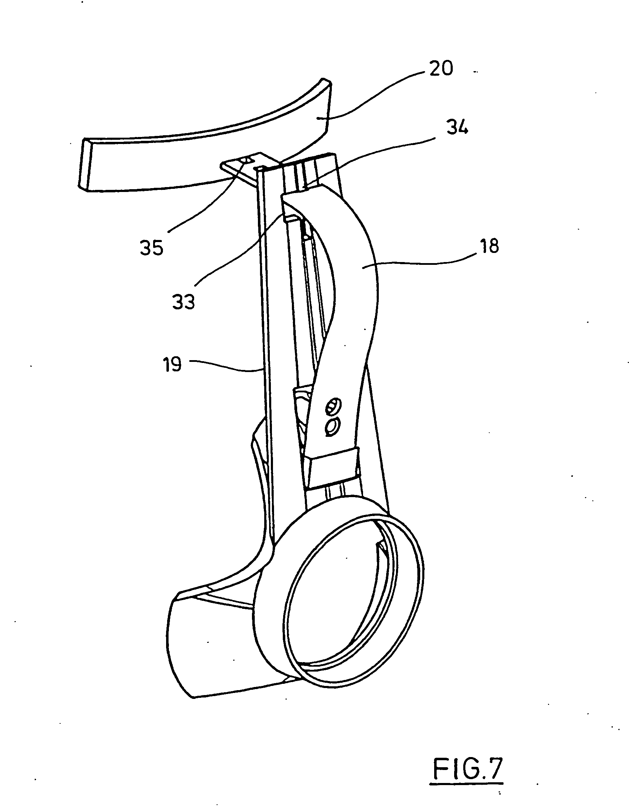

Gemäß der Ausführungsform in Fig. 7 ist das Verbindungselement (18) mit zwei Fixierausnehmungen (36) versehen. Hierdurch ist es möglich, eine jeweilige Fixierpositionierung vorzugeben. Durch diese Vorgebbarkeit ist es möglich, in Abhängigkeit von der Länge des aus der Ausnehmung (33) in Richtung auf die Stirnstütze (20) herausstehenden Endes des Verbindungselementes (18) die Fixierung des der Ausnehmung (33) angewandten Endes des Verbindungselementes (18) derart vorzugeben, daß die Wölbung des Verbindungselementes (18) zwischen der Ausnehmung (33) und der Fixierausnehmung (36) innerhalb eines vorgebbaren Intervalles liegt und insbesondere eine zu starke Durchbiegung vermieden wird.

## Patentansprüche

1. Atemmaske, die einen Maskengrundkörper (13), einen Schlauchanschluß sowie mindestens ein Dichtelement aufweist und die mit einer Stirnstütze (20) versehen ist, die relativ zum Maskengrundkörper (13) positionierbar angeordnet ist, sowie bei der ein die Stirnstütze (20) mit dem Maskengrundkörper (13) koppelndes Verbindungselement (18) relativ zu einer Führung (19) derart verschieblich angeordnet ist, daß das Verbindungselement (18) vorgebbar weit aus der Führung (19) herausragt und relativ zur Führung (19) in mindestens zwei unterschiedlichen Positionierungen arretierbar ist, und ein aus der Führung (19) herausragendes Ende des Verbindungselementes (18) im wesentlichen senkrecht zu einer Grundebene (29) des Maskengrundkörpers (13) verläuft, **dadurch gekennzeichnet, daß** das Verbindungselement (18) flexibel ausgebildet ist.

2. Atemmaske nach Anspruch 1, **dadurch gekennzeichnet, daß** das Verbindungselement (18) bandartig ausgebildet ist.

3. Atemmaske nach Anspruch 1, **dadurch gekennzeichnet, daß** das verbindungselement (18) spiralartig ausgebildet ist.

4. Atemmaske nach Anspruch 1, **dadurch gekennzeichnet, daß** das Verbindungselement (18) als eine mit einem steifen Endstück versehene Gliederkette ausgebildet ist.

5. Atemmaske nach einem der Ansprüche 1 bis 4, **dadurch gekennzeichnet, daß** die Führung (19) eine gekrümmte Führungsbahn für das Verbindungselement (18) aufweist.

6. Atemmaske nach einem der Ansprüche 1 bis 5, **dadurch gekennzeichnet, daß** ein Arretierelement zur Arretierung des Verbindungselementes (18) relativ zur Führung (19) mindestens einen Vorsprung (34) und mindestens eine Ausnehmung (35) aufweist.

7. Atemmaske nach einem der Ansprüche 1 bis 6, **dadurch gekennzeichnet, daß** das Verbindungselement (18) eine Bedientaste (23) aufweist.

8. Atemmaske nach einem der Ansprüche 1 bis 7, **dadurch gekennzeichnet, daß** das Verbindungselement (18) relativ zur Führung (19) durch eine federnde Verspannung arretierbar ist.

9. Atemmaske nach einem der Ansprüche 1 bis 8, **dadurch gekennzeichnet, daß** das Verbindungselement (18) zwei relativ zueinander im wesentlichen parallel verlaufende Bänder (24, 25) aufweist.

10. Atemmaske nach Anspruch 9, **dadurch gekennzeichnet, daß** eines der Bänder (24, 25) zur Führung und das andere der Bänder (24, 25) zur Abstützung des Verbindungselementes (18) ausgebildet ist.

11. Atemmaske nach einem der Ansprüche 1 bis 10, **dadurch gekennzeichnet, daß** das Verbindungselement (18) im Bereich seines der Stirnstütze (20) abgewandten Endes mindestens eine an einen Fixierzapfen (37) angepaßte Fixierausnehmung (36) aufweist.

12. Atemmaske nach Anspruch 11, **dadurch gekennzeichnet, daß** das Verbindungselement (18) mindestens zwei Fixierausnehmungen (36) aufweist.

## Claims

1. Respiratory mask comprising a mask base body (13), a flexible pipe connector and at least one sealing element, and which is provided with an end support (20) disposed so that it can be positioned relative to the mask base body (13), and a connecting element (18) coupling the end support (20) with the mask base body (13) is disposed so that it can be displaced relative to a guide (19) so that the connecting element (18) extends out of the guide (19) to a pre-definable degree and can be locked in at least two different positions relative to the guide (19), and an end of the connecting element (18) extending out of the guide (19) extends substantially perpendicular to a base plane (29) of the mask base body (13), **characterised in that** the connecting element (18) is of a flexible design.

2. Respiratory mask as claimed in claim 1, **characterised in that** the connecting element (18) is of a strip-shaped design.

3. Respiratory mask as claimed in claim 1, **characterised in that** the connecting element (18) is of a spiral-type design.

4. Respiratory mask as claimed in claim 1, **characterised in that** the connecting element (18) is provided in the form of a link chain with a rigid end piece.

5. Respiratory mask as claimed in one of claims 1 to 4, **characterised in that** the guide (19) has a curved guide track for the connecting element (18).

6. Respiratory mask as claimed in one of claims 1 to 5, **characterised in that** a locking element for locking the connecting element (18) relative to the guide (19) has at least one projection (34) and at least one cut-out (35).

7. Respiratory mask as claimed in one of claims 1 to 6, **characterised in that** the connecting element (18) has a control button (23).

8. Respiratory mask as claimed in one of claims 1 to 7, **characterised in that** the connecting element (18) can be locked relative to the guide (19) by means of a resilient clamping action.

9. Respiratory mask as claimed in one of claims 1 to 8, **characterised in that** the connecting element (18) has two strips (24, 25) extending essentially parallel with one another.

10. Respiratory mask as claimed in claim 9, **characterised in that** one of the strips (24, 25) is provided for guiding purposes and the other of the strips (24, 25) is provided as a means of supporting the connecting element (18).

11. Respiratory mask as claimed in one of claims 1 to 10, **characterised in that** the connecting element (18) has at least one fixing cut-out (36) adapted to a fixing pin (37) in the region of its end remote from the end support (20).

12. Respiratory mask as claimed in claim 11, **characterised in that** the connecting element (18) has at least two fixing cut-outs (36).

## Revendications

1. Masque respiratoire qui présente un corps de base de masque (13), un raccord de tuyau souple, ainsi qu'au moins un élément d'étanchéité, et qui est pourvu d'un appui pour le front (20) qui peut être positionné par rapport au corps de base de masque (13), et dans lequel est disposé un élément de liaison (18), qui relie l'appui pour le front (20) au corps de base de masque (13) et qui peut être déplacé par rapport à un dispositif de guidage (19) de sorte que l'élément de liaison (18) fasse saillie hors du dispositif de guidage (19) d'une longueur pouvant être prédéterminée et puisse être bloqué par rapport au dispositif de guidage (19) dans au moins deux positions différentes, et une extrémité de l'élément de liaison (18), qui fait saillie hors du dispositif de guidage (19), s'étendant sensiblement perpendiculairement par rapport à un plan de base (29) du corps de base de masque (13), **caractérisé en ce que** l'élément de liaison (18) est flexible.

2. Masque respiratoire selon la revendication 1, **caractérisé en ce que** l'élément de liaison (18) est réalisé en forme de bande.

3. Masque respiratoire selon la revendication 1, **caractérisé en ce que** l'élément de liaison (18) est réalisé en forme de spirale.

4. Masque respiratoire selon la revendication 1, **caractérisé en ce que** l'élément de liaison (18) est réalisé en forme de chaîne à maillons pourvue d'une pièce d'extrémité rigide.

5. Masque respiratoire selon l'une des revendications 1 à 4, **caractérisé en ce que** le dispositif de guidage (19) présente une voie de guidage en courbe pour l'élément de liaison (18).

6. Masque respiratoire selon l'une des revendications 1 à 5, **caractérisé en ce qu'**un élément de blocage pour le blocage de l'élément de liaison (18) par rapport au dispositif de guidage (19) présente au moins une saillie (34) et au moins un évidement (35).

7. Masque respiratoire selon l'une des revendications 1 à 6, **caractérisé en ce que** l'élément de liaison (18) présente un bouton de commande (23).

8. Masque respiratoire selon l'une des revendications 1 à 7, **caractérisé en ce que** l'élément de liaison (18) peut être bloqué par rapport au dispositif de guidage (19) par tension élastique.

9. Masque respiratoire selon l'une des revendications 1 à 8, **caractérisé en ce que** l'élément de liaison (18) présente deux bandes (24, 25) qui s'étendent sensiblement parallèlement l'une par rapport à l'autre.

10. Masque respiratoire selon la revendication 9, **caractérisé en ce que** l'une des bandes (24, 25) est conçue pour le guidage et l'autre bande (24, 25) pour le support de l'élément de liaison (18).

11. Masque respiratoire selon l'une des revendications 1 à 10, **caractérisé en ce que** l'élément de liaison (18) présente, dans la région de son extrémité opposée à son appui pour le front (20), au moins un évidement de fixation (36) adapté à une broche de fixation (37).

12. Masque respiratoire selon la revendication 11, **caractérisé en ce que** l'élément de liaison (18) présente au moins deux évidements de fixation (36).
